# FASCICULE DE BREVET EUROPEEN

(11) **EP 1 332 242 B1**
(45) Date de publication et mention de la délivrance du brevet: **17.11.2004**
(21) Numéro de dépôt: 01983632.9
(22) Date de dépôt: 19.10.2001
(51) Int. Cl.: C25B 3/02, C07C 45/36

(54) **PROCEDE ELECTROCHIMIQUE DE TRANSFORMATION SELECTIVE DES COMPOSES ALKYLAROMATIQUES EN ALDEHYDES**
ELEKTROCHEMISCHES VERFAHREN ZUR SELEKTIVEN UMSETZUNG VON ALKYLAROMATISCHEN VERBINDUNGEN ZU ALDEHYDEN
ELECTROCHEMICAL METHOD FOR SELECTIVELY TRANSFORMING ALKYLAROMATIC COMPOUNDS INTO ALDEHYDES

(30) Priorité: 20.10.2000 FR 0013469
(43) Date de publication de la demande: 06.08.2003
(73) Titulaire: ELECTRICITE DE FRANCE, 75008 Paris (FR)
(72) Inventeur: TREVIN, Stéphane, F-69210 SAINT BEL (FR); SAVALL, André, F-31170 Tournefeuille (FR); BEJAN, Dorin, Brantford, Ontario N3R 6C2 (CA)
(74) Mandataire: Touati, Catherine
(86) Numéro de dépôt international: PCT/FR2001/003247
(87) Numéro de publication internationale: WO 2002/033151

(56) Documents cités:
- FR-A- 2 351 932
- US-A- 4 402 804
- US-A- 6 069 282
- DATABASE WPI Section Ch, Week 199219 Derwent Publications Ltd., London, GB; Class B05, AN 1992-155017 XP002172053 & JP 04 092375 A (TOSOH CORP), 25 mars 1992 (1992-03-25)

## Description

La présente invention concerne un nouveau procédé de transformation sélective des composés alkylaromatiques en aldéhydes correspondants par utilisation d'anodes métalliques et de l'autoxydation par oxygène.

Les aldéhydes aromatiques sont des produits indispensables pour la synthèse d'intermédiaires ou de produits finis à haute valeur ajoutée, notamment dans les industries pharmaceutiques, alimentaires et de la parfumerie.

Ces aldéhydes aromatiques sont obtenus par oxydation catalytique de composés alkylaromatiques.

Il existe plusieurs tendances économiques dans l'industrie chimique actuelle qui ont stimulé le développement de l'oxydation catalytique :
- l'orientation vers des matières premières peu coûteuses ;
- la préoccupation écologique ;
- la nécessité d'augmenter la sélectivité et la cinétique de la réaction ;
- la demande croissante de voies réactionnelles plus simples ;
- le développement de la chimie fine.

L'oxydation sélective de composés organiques, par l'utilisation de l'oxygène moléculaire représente une potentialité importante pour l'industrie chimique. Le développement continu de nouveaux procédés ou de procédés améliorés, qui utilisent l'oxygène moléculaire est favorisé, d'une part, par l'abondance et le prix relativement réduit de ce réactif, et d'autre part, par son avantage du point de vue environnemental.

L'oxygène pur est actuellement l'agent d'oxydation le moins cher. En comparaison, le chlore, largement utilisé dans l'industrie, a un prix plus élevé et son utilisation conduit à des sous produits nécessitant des investissements supplémentaires pour leur traitement avant le rejet dans l'environnement. Le peroxyde d'hydrogène, dont le comportement vis-à-vis de l'environnement est très satisfaisant, est par contre beaucoup plus cher que l'oxygène et le chlore.

L'oxydation sélective des toluènes substitués en aldéhydes aromatiques correspondants est une réaction importante pour la synthèse organique industrielle. Les benzaldéhydes substitués sont des produits de haute valeur ajoutée utilisés comme composants ou intermédiaires dans l'industrie pharmaceutique et en parfumerie. Bien que les aldéhydes correspondants soient des intermédiaires réactionnels, ils ne peuvent être obtenus avec une bonne sélectivité que pour des faibles valeurs de la conversion du substrat. Ceci est dû à la cinétique très rapide de la réaction d'autoxydation des aldéhydes en présence d'oxygène.

A titre d'exemple, le procédé AMOCO MC, initialement mis au point pour la production de l'acide téréphtalique, fait appel aux sels de cobalt et de bromures en milieu acide acétique pour produire des aldéhydes qui ne sont que des intermédiaires de la réaction catalytique. L'isolement des aldéhydes est complexe et doit se faire en continu pour éviter leur oxydation supplémentaire en acide. La formation d'aldéhyde est donc directe mais non sélective.

En raison des difficultés des méthodes purement chimiques, on s'est rapidement orienté vers l'utilisation de méthodes électrochimiques.

La production électrochimique des aldéhydes aromatiques est actuellement assurée par des procédés directs ou indirects utilisant les voies catalytique ou des oxydations multiélectroniques conduisant à de nombreux sous produits.

Dans un procédé direct, l'oxydation des substrats organiques a lieu à l'anode avec la formation d'espèces pouvant être chargées positivement (radicaux cations ou dications). Ces espèces participent ensuite à des réactions avec des nucléophiles et/ou des bases présents dans l'électrolyte. Il est possible que les composés obtenus participent ensuite à d'autres réactions électrochimiques ou à des étapes de transfert homogène d'électrons.

Un exemple est le procédé de la Société BASF qui utilise l'oxydation directe à quatre électrons par molécule de toluène pour former l'aldéhyde correspondant par réaction avec le solvant comme donneur d'oxygène. Les rendements sont cependant faibles car il y a passage par un intermédiaire réactionnel qui est l'acétate du dérivé alcoolique résultant de la première oxydation à deux électrons.

Il est à noter que l'aldéhyde aromatique n'est obtenu qu'après passage par un acétate intermédiaire dans ce procédé. L'aldéhyde aromatique est obtenu de façon sélective, mais il n'est pas obtenu directement.

Dans un procédé indirect, l'anode a, dans une première étape, pour fonction de préparer ou de régénérer en continu l'oxydant. Dans la seconde étape, l'oxydant réagit avec le substrat organique suivant une réaction similaire à celle qui a lieu lorsque l'agent d'oxydation est obtenu par d'autres voies.

Un médiateur peut être déposé sur l'anode comme dans tous les procédés BASF qui utilisent une électrode de graphite recouverte par du Cr₂O₃ dans le but d'améliorer les rendements en aldéhyde comme décrit dans les brevets allemands DE 2855508 et DE 3132726. Pourtant, la consommation d'électricité reste élevée et dépasse la quantité théorique.

Des procédés indirects, qui utilisent un médiateur dissous dans le volume de solution, ont aussi été proposés dans l'industrie pour synthétiser des aldéhydes aromatiques, comme par exemple l'oxydation au cérium (IV) ou l'oxydation au sulfate manganique ; le cérium (IV) ou le sulfate manganique étant régénérés électrochimiquement. Toutefois, si les résultats sont variables (rendements de 27 à 80% pour des sélectivités moyennes), les procédés passent tous par la formation d'un intermédiaire à deux électrons (acétate, monoacétal ou alcool) et cette cinétique est lente et conduit à des sous produits, surtout pour des taux de conversion élevés.

Le brevet US 4 402 804 (PPG Industries) décrit un procédé électrochimique permettant de synthétiser des aldéhydes aromatiques, des alcools aromatiques et des acides aromatiques. La mise en oeuvre de ce procédé aboutit à un mélange d'aldéhydes, d'alcools et d'acides aromatiques et ne permet manifestement pas une obtention sélective de l'aldéhyde. De plus, ce document ne précise aucune température de réaction, pourtant donnée essentielle de la cinétique réactionnelle.

Il existe donc un réel besoin en un procédé moins polluant pour l'environnement, moins coûteux, tant en énergie qu'en réactifs, sélectif, et présentant des rendements élevés.

Forte de ce constat, la Demanderesse a donc cherché à mettre au point un procédé répondant à ces exigences.

Il est du mérite de la Demanderesse d'avoir réussi, contre toutes attentes, après avoir mené une recherche approfondie sur le sujet, à mettre au point un procédé moins polluant pour l'environnement moins coûteux, tant en énergie qu'en réactifs, sélectif, direct, et présentant des rendements élevés.

La présente invention porte donc sur un procédé électrochimique, de transformation sélective de composés alkylaromatiques en aldéhydes aromatiques correspondants, caractérisé par le fait qu'on réalise une oxydation desdits composés alkylaromatiques en phase liquide sur anode métallique et en présence d'un coagent d'oxydation donneur d'oxygène.

Le procédé selon l'invention fait ainsi intervenir deux agents d'oxydation : l'électron et le donneur d'oxygène.

On entend par composés alkylaromatiques, les composés possédant au moins un noyau aromatique ou hétéroaromatique substitué ou non et qui porte au moins une chaîne latérale méthyle, de préférence que l'on peut activer électrochimiquement.

Le noyau aromatique préféré est le benzène.

Les composés alkylaromatiques préférés sont le toluène, le *o*-xylène, le *m*-xylène, le *p*-xylène ou le mésitylène.

En plus de la (ou des) chaîne(s) latérale(s) méthyle(s), les composés alkylaromatiques selon l'invention peuvent porter un ou plusieurs autres substituants, linéaires ou branchés, identiques ou différents.

Les substituants préférés sont les radicaux acyles, acylamino, acyloxy, alkenyles, alkoxy, alkoxyalkyles, alkoxyacyles, alkyles, alkynyles, amino, aminoacyles, aryles, aryalkyles, arylamino, carboxy, carboxyalkyles, cyano, cycloalkyles, cyclohétéroalkyles, halo, hétéroatomes, dont O, N, S, nitro, oxo, thio, ces substituants pouvant eux-mêmes être substitués ou non.

Les substituants alkyles préférés sont les alkyles de C₁ à C₁₅.

Les substituants alkoxy préférés sont MeO, EtO, PrO, BuO.

Les composés alkylaromatiques substitués préférés sont : le *tert*.-butyl-2-méthyl-5-anisole, le *tert*.-butyl-4-toluène, le *tert*.-butyl-5m-xylène et le méthyl-4-anisole.

Différents donneurs d'oxygène peuvent être utilisés dans le procédé selon l'invention, seuls ou en combinaison. De préférence, le donneur d'oxygène est choisi parmi les éthers couronne, l'ozone, le peroxyde d'hydrogène (eau oxygénée), ou le dioxygène de l'air ou le dioxygène pur. Chacun de ces donneurs d'oxygène se comportent très bien vis à vis de l'environnement en n'engendrant pas de sous-produits contenant des sels. Plus préférentiellement encore, on utilise le dioxygène pur en raison de son faible coût et des rendements obtenus.

Le procédé selon l'invention va maintenant être exposé plus en détail.

Dans le procédé selon l'invention, on oxyde des composés alkylaromatiques substitués comme décrits précédemment, en phase liquide et sur anode métallique, ce qui produit des radicaux alkylaromatiques.

Les radicaux alkylaromatiques produits sont dans un premier temps adsorbés sur l'anode. Ils peuvent se solubiliser dans le milieu réactionnel selon une réaction réversible.

A ce stade, les espèces radicalaires produites peuvent participer à des réactions avec les différents nucléophiles et/ou les différentes bases présents dans le milieu réactionnel.

Dans le milieu, plusieurs réactions entrent ainsi en compétition.

Dans l'une de ces réactions, on fait réagir le donneur d'oxygène, qu'on introduit dans le milieu réactionnel, avec les radicaux alkylaromatiques adsorbés et/ou désorbés (en solution), ce qui va mener à l'obtention de l'alcool et de l'aldéhyde correspondant après réarrangement moléculaire.

La Demanderesse a mis en évidence que c'est principalement cette dernière réaction qui est privilégiée par rapport aux autres puisque les réarrangements moléculaires conduisent directement à l'aldéhyde, ce qui minimise les sous produits. Le procédé selon l'invention permet ainsi l'obtenir de rendements au-delà de 65%, avec une bonne sélectivité pour l'aldéhyde. En comparaison, certains des procédés actuels ne présentent qu'un rendement de 50%.

La Demanderesse a de plus découvert que la cinétique de la réaction avec les radicaux alkylaromatiques désorbés est plus rapide que celle de la réaction avec les radicaux alkylaromatiques adsorbés. Les radicaux alkylaromatiques désorbés sont en solution, ce qui augmente la cinétique de réaction avec le donneur d'oxygène. Dans le procédé selon l'invention, on utilisera donc de manière avantageuse une anode permettant la désorption des radicaux formés à sa surface.

Des anodes appropriées dans le procédé selon l'invention sont ainsi des anodes en matériau métallique ou métallique composite qui favoriseront la désorption des radicaux formés à leur surface. Le métal peut être notamment choisi parmi le nickel, le platine, le titane, le plomb ou l'inox.

Des anodes métalliques particulièrement appropriées pour mettre en oeuvre l'invention sont choisies parmi les électrodes DSA (Dimensionally Stable Anode). Ces anodes, souvent utilisées dans l'industrie pour leur durée de vie importante, possèdent une couche de passivation conductrice en surface, ce qui les préserve de la solubilisation. On pourra par exemple utiliser une anode de titane recouverte d'une couche de platine.

Dans un mode de réalisation avantageux du dispositif permettant de mettre en oeuvre le procédé selon l'invention, l'anode et la cathode sont concentriques et séparées par une membrane à diffuseur gazeux, ce qui permet d'introduire de l'oxygène au coeur du milieu réactionnel.

De façon inattendue par rapport à la théorie, les inventeurs ont aussi découvert que l'alcool obtenu par réaction avec le donneur d'oxygène peut être oxydé à son tour sur l'anode et par le donneur d'oxygène pour conduire à l'aldéhyde correspondant. En effet, il est théoriquement très difficile de prévoir cette réaction en raison de la complexité des réactions pouvant survenir et de la diversité des espèces en solution dans le milieu réactionnel.

Dans le procédé selon l'invention, le milieu d'oxydation est en phase liquide. On peut utiliser tous les solvants qui ne laissent pas de résidus capables d'interagir avec les espèces en solution pour donner des sous produits. En raison de la différence de potentiel appliquée, le milieu de réaction comprend préférablement au moins un solvant qui peut être choisi parmi ceux qui possèdent une bonne permittivité diélectrique, comme par exemple le méthanol, l'éthanol, l'eau ou encore les acides de faible poids moléculaire ayant une masse molaire inférieure ou égale à 150 g/mol comme l'acide acétique (AcOH), l'acide méthanesulfonique (CH₃SO₃H), l'acide triflique (CF₃CO₂H), l'acide sulfurique (H₂SO₄) ou/et l'acide hydrogenofluorosulfonique (HSO₃F).

On peut aussi utiliser par exemple une coupe d'acides de faible poids moléculaire ou des mélanges des composés cités ci-dessus, avec de l'eau, de l'éthanol et/ou du méthanol ou des mélanges entre acides, comme notamment AcOH/(H₂O), H₂SO₄/(H₂O), H₂SO₄/(C₂H₅OH), HSO₃F/(AcOH), C₂H₅OH/(H₂O), CH₃OH/(H₂O).

La présence d'eau permet de préserver l'anode par oxydation superficielle protectrice de sa surface.

Selon un mode de réalisation du procédé selon l'invention, on peut utiliser un milieu réactionnel anhydre avec comme solvant les mélanges H₂SO₄/(C₂H₅OH) ou HSO₃F/(AcOH). Ceci permet d'utiliser un solvant possédant un plus grand domaine d'électroactivité et permet donc aussi de minimiser la réaction secondaire d'oxydation du solvant. En outre, dans ce cas, on ne favorise pas la formation électrochimique directe de l'alcool correspondant au composé aromatique substitué.

Il est à noter que la réaction d'oxydation de l'alcool, éventuellement formé, en aldéhyde a tout de même lieu dans ce cas. Le procédé selon l'invention démontre donc aussi sa valeur dans ce cas précis.

Selon un autre mode de réalisation du procédé selon l'invention, on peut utiliser un ou plusieurs électrolytes supports pour augmenter la conductivité et conduire l'électrolyse. Ces électrolytes supports peuvent notamment être des acétates de sodium (AcONa) ou de potassium (AcOK), des tétrafluoroborates de sodium (BF₄Na), de tetraethylamine (BF₄NEt₄) ou de tetrabutylamine ((BF₄NBu₄), des trifluorométhanesulfonates de tetraethylamine (CF₃SO₃NEt₄) ou de tetrabutylamine (CF₃SO₃NBu₄) ou encore des fluorosulfonates de sodium. (FSO₃Na) ou de potassium (FSO₃K). La concentration d'électrolyte support est de préférence comprise entre 10⁻² et 1 M.

Le procédé selon l'invention de la Demanderesse présente de nombreux avantages.

Grâce au procédé selon l'invention la consommation d'énergie est diminuée de moitié, ce qui représente une réduction importante de coût. En effet, la transformation en aldéhyde ne nécessite que deux électrons par molécule dans le procédé selon l'invention, contre quatre par molécule, pour les procédés actuellement utilisés.

Le procédé selon l'invention est en outre deux à trois fois plus rapide que ceux actuellement utilisés.

L'utilisation du dioxygène, l'un des oxydants les moins cher sur le marché permet aussi une réduction des coûts. Il a aussi l'avantage d'être non polluant et de ne pas donner des sous-produits contenant des sels qu'il faudrait traiter ultérieurement.

De plus, dans le procédé selon l'invention la réaction peut avoir lieu à des températures relativement basses, de l'ordre de 60 à 100°C, dans une cellule classique, adaptée à un traitement électrochimique et à des pressions moins importantes que celles mises en oeuvres dans l'art antérieur. Un dispositif permettant la mise en oeuvre du procédé selon l'invention peut être par exemple un réacteur de Grignard à double enveloppe avec un filtre presse.

Enfin, et de façon tout à fait surprenante, le procédé de la Société Demanderesse permet de synthétiser un produit qui n'a jamais été préparé auparavant : le tert.-butyl-4 méthoxy-3 benzaldéhyde. Ce composé est un intermédiaire dans la synthèse du musc ambrette (tert. Butyl-2 dinitro-4,6 méthyl-5 anisole), produit à haute valeur ajoutée, très prisé par l'industrie cosmétique et dans la parfumerie.

Les exemples qui suivent illustrent la présente invention sans pour autant la limiter.

Les exemples 1 à 3 ont pour objet de démontrer l'influence du matériau anodique et de la présence d'oxygène sur la sélectivité de la réaction lors de la transformation sélective de différents composés aromatiques en aldéhydes correspondants par comparaison aux procédés classiques.

### EXEMPLE 1 : transformation du méthyl-4 anisole (4-MA) en aldéhyde anisique-4.

On réalise trois électrolyses du méthyl-4 anisole (4-MA) à une concentration de 0,2 M, dans l'acide acétique, avec NaOAc comme électrolyte support à 1 M.

Ces trois électrolyses sont conduites dans un réacteur de Grignard, respectivement avec une anode de carbone et sous atmosphère d'azote pour la première, avec une anode de carbone et sous atmosphère d'oxygène pour la seconde, et avec une anode de platine et sous atmosphère d'oxygène pour la troisième qui correspond au procédé selon à l'invention.

La température de réaction est de 80°C, l'intensité/surface de l'anode est de 78 A/m² et la charge est de 1,86 F/mol.

Les rendements des différents produits obtenus lors de ces trois électrolyses se trouvent dans le Tableau 1.

**Tableau 1 :**

| **Procédé** | **Essai 1 Azote /C** | **Essai 2 Oxygène/C** | **Essai 3 Oxygène/Pt** |
|---|---|---|---|
| **4-MA (%)** | 21 | 21 | 7 |
| **Alcool (%)** | 0 | 2 | 9 |
| **Acétate (%)** | 72 | 43 | 12 |
| **Aldéhyde (%)** | 2 | 26 | 64 |
| **Acide (%)** | 0 | 2 | 3 |
| **Acétate /Aldéhyde** | 36 | 1,6 | **0,18** |
| **Sélectivité de l'aldéhyde (%)** | 2,5 | 32,9 | **68,8** |

### EXEMPLE 2 : transformation sélective du tert-butyl-4 toluène (4tBuT) en tert.-butyl-4 benzaldéhyde.

On réalise trois électrolyses du tert-butyl-4 toluène (4tBuT), à une concentration de 0,2 M dans AcOH-H₂O, avec NaBF₄ comme électrolyte support à 0,1 M. La température de réaction est de 80°C, l'intensité/surface de l'anode est de 78 A/m² et la charge est de 1,86 F/mol.

Dans la première électrolyse, on utilise une anode de carbone et de l'azote. Dans la seconde électrolyse, on utilise une anode de carbone et de l'oxygène. Enfin dans la troisième électrolyse, on utilise une anode de platine et de l'oxygène conformément au procédé selon l'invention.

Les rendements des différents produits obtenus lors de ces trois électrolyses sont répertoriés dans le Tableau 2.

**Tableau 2 :**

| **Procédé** | **Essai 1 Azote /C** | **Essai 2 *Oxygène/C** | **Essai 3 Oxygène/Pt** |
|---|---|---|---|
| **4tBuT (%)** | 41 | 17 | 35 |
| **Alcool (%)** | 0 | 0 | 0 |
| **Acétate (%)** | 53 | 43 | 11 |
| **Aldéhyde (%)** | 4 | 28 | 48 |
| **Acide (%)** | 0 | 4 | 6 |
| **Acétate /Aldéhyde** | 13 | 1,5 | **0,23** |
| **Sélectivité de l'aldéhyde (%)** | 6,7 | 33,7 | **73,8** |

| | | | |
|---|---|---|---|
| * dans AcOH/Et₄NBF₄ à 0,1 M et i=63 A/m². | | | |

### EXEMPLE 3 : transformation sélective du tert.-butyl-5 m-xylène (5tBumX) en tert. Butyl-5 méthyl-3 benzaldéhyde

On réalise deux électrolyses d'une solution de tert.-butyl-5 m-xylène à 0,2 M dans AcOH-H₂O ; l'électrolyte support étant NaBF₄ à 0,1 M, la température étant de 80°C, l'intensité surfacique étant de 78 A/m² et la charge étant de 2,8 F/mol.

Dans la première électrolyse, on utilise une anode de carbone et de l'azote. Dans la deuxième électrolyse, on utilise un anode de platine et de l'oxygène conformément au procédé selon l'invention.

Les rendements des différents produits à l'issue de ces trois électrolyses, sont présentés dans le tableau 3.

**Tableau 3 :**

| **Procédé** | **Essai 1 Azote /C** | **Essai 2 Oxygène/Pt** |
|---|---|---|
| **5tBumX (%)** | 18 | 11 |
| **Alcool (%)** | 0,5 | 3 |
| **Acétate (%)** | 67 | 12 |
| **Aldéhyde (%)** | 8 | 61 |
| **Acide (%)** | 0 | 6 |
| **Acétate /Aldéhyde** | 8 | **0,19** |
| **Sélectivité de l'aldéhyde (%)** | 9,7 | **68,5** |

Ces trois exemples montrent que le procédé selon l'invention permet d'avoir un rapport Acétate/Aldéhyde inférieur à 0,25, ce qui signifie que le rendement d'aldéhyde est plus de quatre fois supérieur à celui d'acétate.

### EXEMPLE 4

On réalise deux électrolyses sous azote du tert-butyl-4 toluène à 0,2 M dans AcOH-H₂O, à une température de 80 °C et avec une charge de 2,05 F/mol.

Dans la première électrolyse, on utilise une anode de carbone et de l'azote avec NaOAc comme électrolyte support.

Dans la deuxième électrolyse, on utilise une anode de platine et de l'oxygène conformément au procédé de l'invention avec Et₄NBF₄ comme électrolyte support.

Le but de ces deux expériences est de démontrer l'influence de l'électrolyte sur la première étape électrochimique (E₁) du mécanisme réactionnel.

Les rendements des différentes espèces chimiques présentes à l'issue de ces électrolyses sont présentés dans le Tableau 4.

**Tableau 4 :**

| **Procédé** | **Essai 1 Azote /C** | **Essai 2 Oxygène/Pt** |
|---|---|---|
| **4tBuT (%)** | 90 | 5 |
| **Alcool (%)** | 0 | 0 |
| **Acétate (%)** | 4 | 86 |
| **Aldéhyde (%)** | 1 | 3 |
| **Acide (%)** | 0 | 0 |

## Revendications

1. Procédé électrochimique de transformation sélective de composés alkylaromatiques en aldéhydes aromatiques correspondants, **caractérisé par le fait qu'**on réalise une oxydation en phase liquide à une température comprise entre 60 et 100°C desdits composés alkylaromatiques, sur anode métallique et en présence d'un coagent d'oxydation donneur d'oxygène.

2. Procédé selon la revendication 1 dans lequel les composés alkylaromatiques sont choisis dans le groupe comprenant les composés possédant au moins un noyau aromatique ou hétéroaromatique éventuellement substitué ou non et qui porte au moins une chaîne latérale méthyle tels que notamment le toluène, le *o*-xylène, le *m*-xylène, le *p*-xylène ou le mésitylène.

3. Procédé selon l'une ou l'autre des revendications 1 ou 2 dans lequel le coagent d'oxydation donneur d'oxygène est choisi dans le groupe comprenant les éthers couronne, l'ozone, le peroxyde d'hydrogène, le dioxygène de l'air, le dioxygène pur et leurs mélanges.

4. Procédé selon l'une quelconque des revendications 1 à 3 dans lequel l'anode métallique est choisie dans le groupe comprenant des anodes dont au moins une partie de la surface comprend du nickel, du platine, du titane, du plomb, de l'inox ou de leurs alliages, des anodes DSA ou des anodes métallique-composite.

5. Procédé selon l'une quelconque des revendications 1 à 4 dans lequel le milieu réactionnel comprend au moins un solvant choisi dans le groupe comprenant les solvants qui possèdent une bonne permittivité diélectrique.

6. Procédé selon l'une quelconque des revendications 1 à 5 dans lequel le solvant est choisi dans le groupe comprenant le méthanol, l'éthanol, l'eau ou les acides, de préférence les acides de poids moléculaire inférieur ou égal à 150g/mol, et plus préférentiellement encore l'acide acétique (AcOH), l'acide méthanesulfonique (CH₃SO₃H), l'acide triflique (CF₃CO₂H), l'acide sulfurique (H₂SO₄) et l'acide hydrogenofluorosulfonique (HSO₃F) ou leurs mélanges.

7. Procédé selon l'une quelconque des revendications 1 à 6 dans lequel le milieu comprend un électrolyte support choisi notamment dans le groupe comprenant l'acétate de sodium (AcONa) ou de potassium (AcOK), le tétrafluoroborate de sodium (BF₄Na), de tétraéthylamine (BF₄NEt₄) ou de tétrabutylamine ((BF₄NBu₄), le trifluorométhanesulfonate de tétraéthylamine (CF₃SO₃NEt₄) ou de tétrabutylamine (CF₃SO₃NBu₄) et le fluorosulfonate de sodium (FSO₃Na) ou de potassium (FSO₃K), la concentration dudit électrolyte étant de préférence comprise entre 10⁻² et 1 M.

8. Procédé selon l'une quelconque des revendications 1 à 7 pour la transformation sélective du tert.-butyl-2 méthyl-5 anisole en tert.-butyl-4 méthoxy-3 benzaldéhyde.

9. Procédé selon l'une quelconque des revendications 1 à 8 pour la transformation sélective du tert.-butyl-4 toluène, du tert.-butyl-5 m-xylène ou du méthyl-4 anisole respectivement en tert.-butyl-4 benzaldéhyde, en tert. Butyl-5 méthyl-3 benzaldéhyde ou en aldéhyde anisique-4.

10. Tert.-butyl-4 méthoxy-3 benzaldéhyde.

## Patentansprüche

1. Elektrochemisches Verfahren zur selektiven Umlagerung von alkylaromatischen Verbindungen in entsprechende aromatische Aldehyde, **dadurch gekennzeichnet, dass** eine Oxidation in flüssiger Phase bei einer Temperatur zwischen 60 und 100°C der alkylaromatischen Verbindungen auf einer Metallanode sowie in Anwesenheit eines Sauerstoffbildenden Oxidationskoagenses vorgenommen wird.

2. Verfahren nach Anspruch 1, wobei die alkylaromatischen Verbindungen aus der Gruppe ausgewählt werden, die Verbindungen umfassen, welche zumindest einen aromatischen oder heteroaromatischen Kern umfassen, der gegebenenfalls substituiert oder nicht substituiert ist, und der zumindest eine laterale Methylkette trägt, wie insbesondere Toluol, o-Xylol, m-Xylol, p-Xylol oder Mesitylen.

3. Verfahren nach Anspruch 1 oder 2, bei welchem das Sauerstoff-bildende Oxidationskoagens aus der Gruppe ausgewählt ist, welche Ring-Ether, Ozon, Wasserstoffperoxid, Luftdioxid, reines Dioxid und Gemische hieraus enthält.

4. Verfahren nach einem der Ansprüche 1 bis 3, bei welchem die Metallanode ausgewählt ist aus der Gruppe, Anoden umfasst, von denen zumindest ein Teil der Oberfläche Nikkel, Platin, Titan, Blei, Edelstahl oder Legierungen hieraus enthält, DSA-Anoden oder Metall-Verbundstoffanoden.

5. Verfahren nach einem der Ansprüche 1 bis 4, bei dem das Reaktionsmedium zumindest ein Lösungsmittel umfasst, das aus der Gruppe ausgewählt ist, welche Lösungsmittel umfasst, die eine gute dielektrische Permittivität besitzen.

6. Verfahren nach einem der Ansprüche 1 bis 5, bei dem das Lösungsmittel aus der Gruppe ausgewählt ist, die Methanol, Ethanol, Wasser oder Säuren umfasst, bevorzugt Säuren mit einem Molekulargewicht, das kleiner oder gleich 150 g/mol ist, und besonders bevorzugt Essigsäure (AcOH), Methansulfonsäure (CH₃SO₃H), Trifluoressigsäure (CF₃CO₂H), Schwefelsäure (H₂SO₄) und Fluorwasserstoffsulfonsäure (HSO₃F) oder Gemische hieraus.

7. Verfahren nach einem der Ansprüche 1 bis 6, bei dem das Medium einen Träger-Elektrolyten umfasst, der insbesondere ausgewählt ist aus der Gruppe, Natriumacetat (AcONa) oder Kaliumacetat (AcOK), Natriumtetrafluorborat (BF₄Na), Tetraethylamin (BF₄Net₄) oder Tetrabutylamin (BF₄NBu₄), Tetraethylamintrifluormethansulfonat (CF₃SO₃NEt₄) oder Tetrabutylamin (CF₂SO₃NBu₄) sowie Natriumsulfonat (PSO₃Na) oder Kaliumfluorsulfonat (PSO₃K) umfasst, wobei die Konzentration des Elektrolyten bevorzugt zwischen 10⁻² und 1 M beträgt.

8. Verfahren nach einem der Ansprüche 1 bis 7 zur selektiven Umlagerung von tert-Butyl-2-methyl-5-anisol in tert-Butyl-4-methoxy-3-benzaldehyd.

9. Verfahren nach einem der Ansprüche 1 bis 8 zur selektiven Umlagerung von tert-Butyl-4-toluol, tert-Butyl-5-m-xylol oder Methyl-4-anisol in tert-Butyl-4-benzaldehyd, tert-Butyl-5-methyl-3-benzaldehyd oder Aldehydanisin-4.

10. tert-Butyl-4-methoxy-3-benzaldehyd.

## Claims

1. Electrochemical method for the selective conversion of alkylaromatic compounds into the corresponding aromatic aldehydes, **characterized in that** oxidation of the said alkylaromatic compounds is performed in a liquid phase at a temperature of between 60 and 100°C , on a metal anode and in the presence of an oxygen donor oxidation coagent.

2. Method according to claim 1 wherein the alkylaromatic compounds are chosen from the group comprising compounds possessing at least one aromatic or heteroaromatic nucleus possibly substituted or not and which carries at least one methyl side chain such as in particular toluene, o-xylene, m-xylene, p-xylene or mesitylene.

3. Method according to one or other of claims 1 or 2 wherein the oxygen donor oxidation coagent is chosen from the group comprising crown ethers, ozone, hydrogen peroxide, dioxygen from the air, pure dioxygen and mixtures thereof.

4. Method according to any one of claims 1 to 3 wherein the metal anode is chosen from the group comprising anodes of which at least part of the surface comprises nickel, platinum, titanium, lead, stainless steel or alloys thereof, DSA anodes or metal-composite anodes.

5. Method according to any one of claims 1 to 4 wherein the reaction medium includes at least one solvent chosen from the group comprising solvents that possess good dielectric permittivity.

6. Method according to any one of claims 1 to 5 wherein the solvent is chosen from the group comprising methanol, ethanol, water or acids, preferably acids with a molecular weight less than or equal to 150 g/mol, and even more preferably acetic acid (AcOH), methanesulfonic acid (CH₃SO₃H), triflic acid (CF₃CO₂H), sulfuric acid (H₂SO₄) and hydrogenofluorosulfonic acid (HSO₃F) or mixtures thereof.

7. Method according to any one of claims1 to 6, wherein the medium includes a supporting electrolyte chosen in particular from the group comprising sodium acetate (AcONa) or potassium acetate (AcOK), sodium tetrafluoroborate (BF₄Na), tetraethylamine tetrafluoroborate (BF₄NEt₄) or tetrabutylamine tetrafluoroborate (BF₄NBu₄), tetraethylamine trifluoromethanesulfonate (CF₃SO₃NEt₄) or tetrabutylamine trifluoromethanesulfonate (CF₃SO₃NBu₄) and sodium fluorosulfonate (FSO₃Na) or potassium fluorosulfonate (FSO₃K), the concentration of the said electrolyte being preferably between 10⁻² and 1 M.

8. Method according to any one of claims 1 to 7 for the selective conversion of tert-butyl-2-methyl-5 anisole into tert-butyl-4 methoxy-3 benzaldehyde.

9. Method according to any one of claims 1 to 8 for the selective conversion of tert-butyl-4 toluene, tert-butyl-5 m-xylene or methyl-4 anisole respectively into tert-butyl-4 benzaldehyde, tert-butyl-5 methyl-3 benzaldehyde or anisinic aldehyde-4.

10. Tert-butyl-4 methoxy-3 benzaldehyde
